# EUROPÄISCHE PATENTSCHRIFT

(11) **EP 0 463 434 B1**
(45) Veröffentlichungstag und Bekanntmachung des Hinweises auf die Patenterteilung: **28.12.1994**
(21) Anmeldenummer: 91109389.6
(22) Anmeldetag: 07.06.1991
(51) Int. Cl.: C07C 69/54, C07C 67/58

(54) **Verfahren zur Herstellung von monoethylenisch ungesättigten Carbonsäureestern**
Method for the production of monoethylenically unsaturated carboxylic acid esters
Procédé pour la fabrication d'esters d'acides carboxyliques monoéthyléniquement insaturés

(30) Priorität: 21.06.1990 DE 4019781
(43) Veröffentlichungstag der Anmeldung: 02.01.1992
(73) Patentinhaber: BASF Aktiengesellschaft, 67063 Ludwigshafen (DE)
(72) Erfinder: Nestler, Gerhard, Dr., W-6700 Ludwigshafen (DE); Ruckh, Peter, Dr., W-6800 Mannheim 51 (DE); Lazarus, Manfred, W-6907 Nussloch (DE)

(56) Entgegenhaltungen:
- EP-A- 0 202 610
- US-A- 2 917 538
- CHEMICAL ABSTRACTS, vol. 106, 1987, Columbus, Ohio, US, abstract no. 157005k, page 10; M. FUJII et al, "(Meth)acrylate esters"; JP-A-61 243 064

## Beschreibung

Die Vorliegende Erfindung betrifft ein neues verfahren zur Herstellung von monoethylenisch ungesättigten Carbonsäureestern, bei dem man in flüssiger Phase bei Temperaturen von 85 bis 140°C in Gegenwart von wirksamen Mengen an Polymerisationsinhibitoren und einem inerten Schleppmittel für Wasser 3 bis 5 C-Atome enthaltende α,β-ethylenisch ungesättigte Carbonsäuren mit 6 bis 14 C-Atome enthaltenden Alkanolen im Molverhältnis 0,8:1 bis 1,2:1 in Gegenwart von 0,1 bis 5 Gew.-%, bezogen auf das Carbonsäure/Alkanol-Gemisch, Schwefelsäure verestert, nach beendeter Veresterung das Produktgemisch durch Extraktion in eine wäßrige und in eine organische Phase trennt und aus der organischen Phase den gebildeten monoethylenisch ungesättigten Carbonsäureester abtrennt.

Monoethylenisch ungesättigte Carbonsäureester aus 3 bis 5 C-Atome enthaltenden α,β-ethylenisch ungesättigten Carbonsäuren und 6 bis 14 C-Atome enthaltenden Alkanolen sind unter anderem als Monomere zur Herstellung von Polymeren, z.B. für als Klebstoffe geeignete wäßrige Polymerdispersionen, von Bedeutung.

Ullmann's Encyclopedia of Industrial Chemistry, Vol. A1, p. 168-169, 1985, VCH D-6940 Weinheim, betrifft ein verfahren zur Herstellung von Estern aus höheren Alkanolen und Acrylsäure im molaren Verhältnis von 1:1 bis 1,1:1 bei Temperaturen von 85 bis 95°C in flüssiger Phase in Gegenwart von Polymerisationsinhibitoren und Schwefelsäure sowie eines inerten Schleppmittels für Wasser, das dadurch gekennzeichnet ist, daß nach beendeter Veresterung das Produktgemisch mit wäßriger Alkalilösung extrahiert und aus der bei der Extraktion anfallenden organischen Phase der Acrylsäureester abgetrennt wird. Nachteilig an diesem verfahren ist, daß die parallel zur eigentlichen Veresterung aus Schwefelsäure und Alkanol sich bildende Alkylschwefelsäure (die eigentlich katalytisch wirksame Säure) sowie gegegebenenfalls nicht mit Alkanol veresterte Schwefelsäure bei der Extraktion in die entsprechenden, keine Wertverbindungen darstellenden, Alkalimetallsalze überführt werden, die als solche auch nicht für eine weitere Veresterung verwendet werden können, was insbesondere bei einer kontinuierlichen Ausführung des Verfahren zu einem laufenden Verlust an Wertedukt führt. Aus analogem Grund ist das bekannte Verfahren für die Variante der Veresterung in Gegenwart eines Überschusses von Acrylsäure ungeeignet, da bei der Extraktion nicht umgesetzte Acrylsäure in ihr Alkalimetallsalz umgewandelt würde. Ein Überschuß der einen Ausgangskomponente gegenüber der anderen Ausgangskomponente bewirkt bei typischen Gleichgewichtsreaktionen wie Veresterungen eine Erhöhung des Umsatzes der im Unterschuß eingesetzten Komponente.

Aufgabe der vorliegenden Erfindung war daher, ein Verfahren zur Herstellung von monoethylenisch ungesättigten Carbonsäureestern, bei dem man in flüssiger Phase bei erhöhten Temperaturen in Gegenwart von Polymerisationsinhibitoren und einem inerten Schleppmittel für Wasser α,β-ethylenisch ungesättigte Carbonsäuren mit höheren Alkanolen in Gegenwart von Schwefelsäure verestert, nach beendeter Veresterung das Produktgemisch durch Extraktion in eine wäßrige und in eine organische Phase trennt und aus der organischen Phase den gebildeten monothylenisch ungesättigten Carbonsäureester abtrennt, zur Verfügung zu stellen, das die genannten Nachteile mindert.

Demgemäß wurde ein neues Verfahren zur Herstellung von monoethylenisch ungesättigten Carbonsäureestern, bei dem man in flüssiger Phase bei Temperaturen von 85 bis 140°C in Gegenwart von wirksamen Mengen an Polymerisationsinhibitoren und einem inerten Schleppmittel für Wasser 3 bis 5 C-Atome enthaltende, α,β-ethylenisch ungesättigte Carbonsäuren mit 6 bis 14 C-Atome enthaltenden Alkanolen im Molverhältnis 0,8:1 bis 1,2:1 in Gegenwart von 0,1 bis 5 Gew.-%, bezogen auf das Carbonsäure/Alkanol-Gemisch, Schwefelsäure verestert, nach beendeter Veresterung das Produktgemisch durch Extraktion in eine wäßrige und in eine organische Phase trennt und aus der organischen Phase den gebildeten monoethylenisch ungesättigten Carbonsäureester abtrennt, gefunden, das dadurch gekennzeichnet ist, daß man nach beendeter Veresterung
a) das Produktgemisch in einer ersten Extraktionsstufe bei einer Temperatur von 10 bis 40°C mit 5 bis 30 Vol.-%, bezogen auf das Produktgemisch, Wasser extrahiert, und
b) in einer zweiten Extraktionsstufe bei einer Temperatur von 10 bis 30°C die wäßrige Phase aus der ersten Extraktionsstufe mit der ein- bis vierfachen Gewichtsmenge an zu veresterndem Alkanol extrahiert.

Die Extraktion des Produktgemisches mit Wasser kann sowohl in einem, als auch in mehreren Schritten nach dem Mixer-Settler-Prinzip oder nach anderen an sich bekannten Extraktionsweisen erfolgen, wie sie z.B. in Ullmanns Encyklopädie der technischen Chemie, Bd. 2, S. 560-564, 1972, VCH D-6940 Weinheim, beschrieben sind. Die im Produktgemisch enthaltenen Säuren werden bei der Extraktion im überwiegenden Ausmaß von der wäßrigen Phase aufgenommen, wobei der Extraktionsgrad für die Summe aus Alkylschwefelsäure und Schwefelsäure in der Regel 90 bis 99 % beträgt, wohingegen für gegebenenfalls nicht umgesetzte α,β-monoethylenisch ungesättigte Carbonsäuren im allgemeinen Extraktionsgrade von 50 bis 90 % erzielt werden. Die Extraktionstemperatur in der ersten Extraktionsstufe wird vorzugsweise so eingestellt, daß man das Produktgemisch auf 10 bis 60°C abkühlt und mit Wasser extrahiert, das eine Temperatur von 10 bis 30°C aufweist, wodurch normalerweise eine Gemischtemperatur von 10 bis 40°C erzielt wird.

Die Überführung der Säuren, bei der Extraktion der säurehaltigen wäßrigen Phase aus der ersten Extraktionsstufe, in die alkanolische Phase ist in der Regel so bemessen, daß die abschließend in der alkanolischen Phase enthaltene Summe aus Alkylschwefelsäure und Schwefelsäure, bezogen auf die ursprünglich zur Veresterung eingesetzte Schwefelsäuremenge, 80 bis 95 Mol.-% beträgt, wohingegen derselbe Wert für die α,β-monoethylenisch ungesättigten Carbonsäuren, bezogen auf die bei der Veresterung gegebenenfalls nicht umgesetzte α,β-monoethylenisch ungesättigte Carbonsäure, 40 bis 80 Mol.-% beträgt.

Überrraschenderweise treten bei dem erfindungsgemäßen Verfahren weder in der ersten, noch in der zweiten Extraktionsstufe in erkennbarem Ausmaß Nebenreaktionen auf. So wurde in der ersten Extraktionsstufe weder eine Verseifung des Esters noch eine Addition von Wasser an die Doppelbindung beobachtet. Dies ist um so bemerkenswerter, als in Folge der zur Extraktion verwendeten relativ geringen Wassermenge das Extraktionsmedium deutlich sauer ist und die Behandlung von ungesättigten Carbonsäureestern mit wäßrigen Lösungen starker Säuren in der Regel sowohl eine säurekatalysierte Esterspaltung als auch eine säurekatalysierte Addition von Wasser an die Doppelbindung bewirkt.

In entsprechender Weise war in der zweiten Extraktionsstufe sowohl die säurekatalysierte Bildung von Dialkylethern, als auch die säurekatalysierte Bildung von Dialkylsulfaten zu erwarten. Die alkanolische Phase der zweiten Extraktionsstufe, die im wesentlichen die für die eigentliche Veresterung katalytisch wirksamen Schwefelsäuren und gegebenenfalls die nicht umgesetzte α,β-monoethylenisch ungesättigte Carbonsäure enthält, kann daher in vorteilhafter Weise der Veresterungsstufe wieder zugeführt werden und die im wesentlichen nur Wasser enthaltende wäßrige Phase der zweiten Extraktionsstufe eignet sich in vorteilhafter Weise, gegebenenfalls ergänzt durch geringe Mengen an Frischwasser, zur Wiederverwendung in der ersten Extraktionsstufe. Diese Verfahrensweise ist insbesondere dann von Vorteil, wenn das erfindungsgemäße Verfahren kontinuierlich betrieben wird. Selbstverständlich kann das erfindungsgemäße Verfahren auch diskontinuierlich betrieben werden. Zweckmäßigerweise wird bei einer kontinuierlichen Durchführung des erfindungsgemäßen Verfahrens die in der zweiten Extraktionsstufe für die Extraktion eingesetzte Alkanolmenge so bemessen, daß sie die in der Veresterungsstufe für die Veresterung benötigte Alkanolmenge nicht übersteigt. Weiterhin wird bei einer kontinuierlichen Durchführung des erfindungsgemäßen Verfahrens die in der ersten Extraktionsstufe anfallende organische Phase zweckmäßigerweise destillativ in den gebildeten monoethylenisch ungesättigten Carbonsäureester, gegebenenfalls nicht umgesetztes Alkanol, Schleppmittel sowie einen Destillationsrückstand aufgetrennt und das abgetrennte Schleppmittel sowie das gegebenenfalls abgetrennnte Alkanol in vorteilhafter Weise der Veresterungsstufe wieder zugeführt. Wird eine besonders hohe Reinheit der verschiedenen Destillate gewünscht, ist es empfehlenswert, die organische Phase mittels einer verdünnten wäßrigen Alkalimetallhydroxidlösung zu neutralisieren. Der Destillationsrückstand enthält normalerweise die in der Regel in organischen Flüssigkeiten gut löslichen Polymerisationsinhibitoren. Beispiele für geeignete Polymerisationsinhibitoren sind Hydrochinon, 4-Methoxyphenol sowie Phenothiazin, die für sich oder als Gemisch verwendet werden können. Überlicherweise werden dem Veresterungsgemisch sowie den ungesättigten Ester enthaltenden Gemischen ca. 0,01 bis 0,1 Gew.-% an Polymerisationsinhibitoren zugesetzt. Für diesen Zweck kann auch der Destillationsrückstand gegebenenfalls wiederverwendet werden. Als Schleppmittel für Wasser eignet sich insbesondere Cyclohexan. Es können diesbezüglich aber auch Mittel wie chlorierte Kohlenwasserstoffe oder Toluol eingesetzt werden. Der wesentliche Vorteil des erfindungsgemäßen Verfahrens liegt in seiner erhöhten Wirtschaftlichkeit begründet. Dieser Vorteil ist dann besonders stark ausgeprägt, wenn Acryl- oder Methacrylsäure mit 6 bis 10 C-Atome enthaltenden Alkanolen, vorzugsweise 2-Ethylhexanol, verestert werden. Insbesondere, wenn die zu veresternden Säuren im Überschuß eingesetzt werden.

### Beispiel 1

Ein Gemisch aus 360 g Acrylsäure, 790 g n-Decanol, 200 g Cyclohexan, 10 g 97 gew.-%iger Schwefelsäure, 0,4 g Phenothiazin und 0,4 g 4-Methoxyphenol wurde während 3 h zum Sieden erhitzt und dabei gebildetes Wasser als Azeotrop abgetrennt. Es wurde ein Produktgemisch erhalten, das 10,2 g Acrylsäure und 23,4 g Decylschwefelsäure enthielt. Der Umsatz an eingesetztem n-Decanol betrug 99,1 %. Anschließend wurde das 1270 g umfassende Produktgemisch bei 40°C nach dem Mixer-Settler-Prinzip zweimal mit je 150 g Wasser extrahiert und die dabei anfallende wäßrige Phase zweimal mit je 300 g n-Decanol in gleicher Weise behandelt. Die vereinigten Decanolphasen (636 g) enthielten 5,6 g Acrylsäure und 21,2 g Decylschwefelsäure. Die in der ersten Extraktionsstufe anfallende organische Phase enthielt (gaschromatographisch ermittelt), bezogen auf die eingesetzte Menge an n-Decanol, 96 % d. Th. des gewünschten Esters aus Acrylsäure und n-Decanol.

### Beispiel 2

a) Ein Gemisch aus 360 g Acrylsäure, 650 g 2-Ethylhexanol, 200 g Cyclohexan, 5 g 97 gew.-%iger Schwefelsäure, 0,4 g Phenothiazin und 0,4 g Methoxyphenol wurde während 3 h zum Sieden erhitzt und dabei gebildetes Wasser als Azeotrop abgetrennt. Es wurde ein Produktgemisch erhalten, das 8,7 g Acrylsäure und 10,4 g 2-Ethylhexylschwefelsäure enthielt. Der Umsatz an eingesetztem 2-Ethylhexanol betrug 98,7 %. Anschließend wurde das 1110 g umfassende Produktgemisch bei 30°C nach dem Mixer-Settler-Prinzip mit 150 g Wasser extrahiert und die dabei anfallende wäßrige Phase zweimal mit je 225 g 2-Ethylhexanol in gleicher Weise behandelt. Die vereinigten 2-Ethylhexanolphasen (475 g) enthielten 3,9 g Acrylsäure und 9,5 g 2-Ethylhexylschwefelsäure. Die in der ersten Extraktionsstufe anfallende organische Phase enthielt (gaschromatographisch ermittelt), bezogen auf die eingesetzte Menge an 2-Ethylhexanol, 97 % d. Th. des gewünschten Esters aus Acrylsäure und 2-Ethylhexanol.
b) Man verfuhr wie bei a), als 2-Ethylhexanol wurde jedoch die in a) in der zweiten Extraktionsstufe anfallende 2-Ethylhexanolphase, ergänzt durch 195 g 2-Ethylhexanol, eingesetzt. Ferner wurden an Stelle von 5 g Schwefelsäure nur 2 g Schwefelsäure neu zugesetzt und die erste Extraktion des Produktgemisches wurde mit der in a) in der zweiten Extraktionsstufe anfallenden wäßrigen Phase, ergänzt durch 10 g Wasser, ausgeführt. Es wurde ein Produktgemisch erhalten, das 14 g Acrylsäure und 13,2 g 2-Ethylhexylschwefelsäure enthielt. Die nach der Extraktion vereinigten 2-Ethylhexanolphasen enthielten 7,7 g Acrylsäure und 12,1 g 2-Ethylhexylschwefelsäure. Die Ausbeute des gewünschten Esters betrug, bezogen auf die eingesetzte Menge 2-Ethylhexanol, 98,2 % d.Th.

### Beispiel 3

a) Ein Gemisch aus 435 g Methacrylsäure, 650 g 2-Ethylhexanol, 200 g Cyclohexan, 5,6 g 97 gew.-%ige Schwefelsäure, 0,4 g Phenothiazin und 0,4 g Methoxyphenol wurde 4 h zum Sieden erhitzt und dabei gebildetes Wasser als Azeotrop abgetrennt. Es wurde ein Produktgemisch erhalten, das 17,8 g Methacrylsäure und 11,5 g 2-Ethylhexylschwefelsäure enthielt. Der Umsatz an eingesetztem 2-Ethylhexanol betrug 98,2 %. Anschließend wurde das 1196 g umfassende Produktgemisch bei 30°C nach dem Mixer-Settler-Prinzip zweimal mit 100 g Wasser extrahiert und die dabei anfallende wäßrige Phase zweimal mit 200 g 2-Ethylhexanol in gleicher Weise behandelt. Die vereinigten 2-Ethylhexanolphasen (419 g) enthielten 7,3 g Methacrylsäure und 10,5 g 2-Ethylhexylschwefelsäure. Die in der ersten Extraktionsstufe anfallende organische Phase enthielt (gaschromatographisch ermittelt), bezogen auf die eingesetzte Menge an 2-Ethylhexanol, 96,8 % des gewünschten Esters aus Methacrylsäure und 2-Ethylhexanol.
b) Man verfuhr wie bei a), als 2-Ethylhexanol wurde jedoch die in a) in der zweiten Extraktionsstufe anfallende 2-Ethylhexanolphase, ergänzt durch 273 g 2-Ethylhexanol, eingesetzt. Ferner werden anstelle von 5 g Schwefelsäure nur 2 g Schwefelsäure neu zugesetzt und die erste Extraktion des Produktgemisches wurde mit der in a) in der zweiten Extraktionsstufe anfallenden wäßrigen Phase, ergänzt durch 10 g Wasser, ausgeführt. Es wurde ein Produktgemisch erhalten, das 12,9 g Methacrylsäure und 14,4 g 2-Ethylhexylschwefelsäure enthielt. Der Umsatz an eingesetztem 2-Ethylhexanol betrug 98,3 %. Die nach der Extraktion vereinigten 2-Ethylhexanolphasen enthielten 6,2 g Methacrylsäure und 13,3 g 2-Ethylhexylschwefelsäure. Die Ausbeute des gewünschten Esters betrug, bezogen auf die eingesetzte Menge 2-Ethylhexanol, 97,9 % d.Th.

## Patentansprüche

1. Verfahren zur Herstellung von monoethylenisch ungesättigten Carbonsäureestern, bei dem man in flüssiger Phase bei Temperaturen von 85 bis 140°C in Gegenwart von wirksamen Mengen an Polymerisationsinhibitoren und einem inerten Schleppmittel für Wasser 3 bis 5 C-Atome enthaltende, α,β-ethylenisch ungesättigte Carbonsäuren mit 6 bis 14 C-Atome enthaltenden Alkanolen im Molverhältnis 0,8:1 bis 1,2:1 in Gegenwart von 0,1 bis 5 Gew.-%, bezogen auf das Carbonsäure/Alkanol-Gemisch, Schwefelsäure verestert, nach beendeter Veresterung das Produktgemisch durch Extraktion in eine wäßrige und in eine organische Phase trennt und aus der organischen Phase den gebildeten monoethylenisch ungesättigten Carbonsäureester abtrennt, dadurch gekennzeichnet, daß man nach beendeter Veresterung
a) das Produktgemisch in einer ersten Extraktionsstufe bei einer Temperatur von 10 bis 40°C mit 5 bis 30 Vol.-%, bezogen auf das Produktgemisch, Wasser extrahiert, und
b) in einer zweiten Extraktionsstufe bei einer Temperatur von 10 bis 30°C die wäßrige Phase aus der ersten Extraktionsstufe mit der ein- bis vierfachen Gewichtsmenge an zu veresterndem Alkanol extrahiert.

## Claims

1. Process for the preparation of a monoethylenically unsaturated carboxylic ester, in which an α,β-ethylenically unsaturated carboxylic acid of 3 to 5 carbon atoms is esterified with an alkanol of from 6 to 14 carbon atoms in a molar ratio of from 0.8 : 1 to 1.2 : 1 in the liquid phase at from 85 to 140°C in the presence of an effective amount of a polymerization inhibitor and of an inert entraining agent for water and in the presence of from 0.1 to 5% by weight, based on the carboxylic acid/alkanol mixture, of sulfuric acid, the product mixture is separated into an aqueous and an organic phase after the esterification is complete and the resulting monoethylenically unsaturated carboxylic ester is separated off from the organic phase, wherein, after esterification is complete,
a) the product mixture is extracted in a first extraction stage at from 10 to 40°C with from 5 to 30% by volume, based on the product mixture, of water and
b) in a second extraction stage, at from 10 to 30°C, the aqueous phase from the first extraction stage is extracted with from an equal amount by weight to four times the amount by weight of the alkanol to be esterified.

## Revendications

1. Procédé de préparation d'esters d'acides carboxyliques monoéthyléniquement insaturés, conformément auquel on estérifie, à des températures de 85 à 140°C, en phase liquide, en présence de proportions actives d'inhibiteurs de polymérisation et d'un agent d'entraînement pour l'eau inerte, des acides carboxyliques α,β-éthyléniquement insaturés et contenant de 3 à 5 atomes de carbone avec des alcanols contenant de 6 à 14 atomes de carbone, dans le rapport molaire de 0,8:1 à 1,2:1, en présence de 0,1 à 5% en poids, par rapport au mélange acide carboxylique/alcanol, d'acide sulfurique, une fois l'estérification achevée, on sépare le mélange de produits par extraction en une phase aqueuse et en une phase organique et on sépare l'ester de l'acide carboxylique monoéthyléniquement insaturé formé de la phase organique, caractérisé en ce que, une fois l'estérification achevée,
a) on extrait le mélange de produits au cours d'une première étape d'extraction, à une température de 10 à 40°C, par de 5 à 30% en volume d'eau, par rapport au mélange de produits, et
b) au cours d'une seconde étape d'extraction et à une température de 10 à 30°C, on extrait la phase aqueuse provenant de la première étape d'extraction avec une proportion pondérale simple à quadruple d'alcanol à estérifier.
